# EUROPEAN PATENT APPLICATION

(11) **EP 1 591 138 A1**
(43) Date of publication of application: **02.11.2005**
(21) Application number: 05009251.9
(22) Date of filing: 27.04.2005
(51) Int. Cl.: A61M 25/00

(54) **Multi-branched catheter-introducer tube**

(30) Priority: 28.04.2004 JP 2004134003
(71) Applicant: USCI Japan Ltd., X (JP)
(72) Inventor: Horita, Yuki, Kanazawa-shi Ishikawa (JP)
(74) Representative: Kunz, Herbert

(57) **Abstract**

A multi-branched tube (10) has a check valve structure (20), a main conduit (40) adapted to be inserted into a blood vessel (BV) with its distal end, a first port tube (60) leading to an inner chamber (24) in the check valve structure (20), and a second port tube (70) connected to the check valve structure (20). A blood flow stopper balloon (50) is mounted on an outer periphery of the main conduit (40) at a portion closer to its distal end. The main conduit (40) has a first guide bore (42) and a second guide bore (46), which are two separate guide bores. The blood flow stopper balloon (50) is adapted to be inflated/deflated by a pressure of a fluid supplied thereinto through the second port tube (70) and the second guide bore (46). Thrombus debris (TH) or the like in the blood vessel (BV) can be drawn and removed together with the blood through the first port tube (60), the inner chamber (24) and the first guide bore (42).

## Description

### BACKGROUND OF THE INVENTION

The present invention relates to a multi-branched catheter-introducer tube (which is usually referred to as a sheath ihtroducer) used in a percutaneous intravascular treatment with utilization of a catheter.

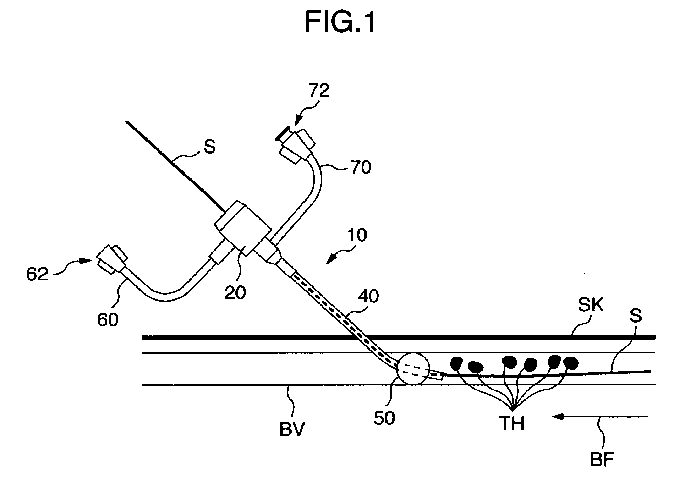

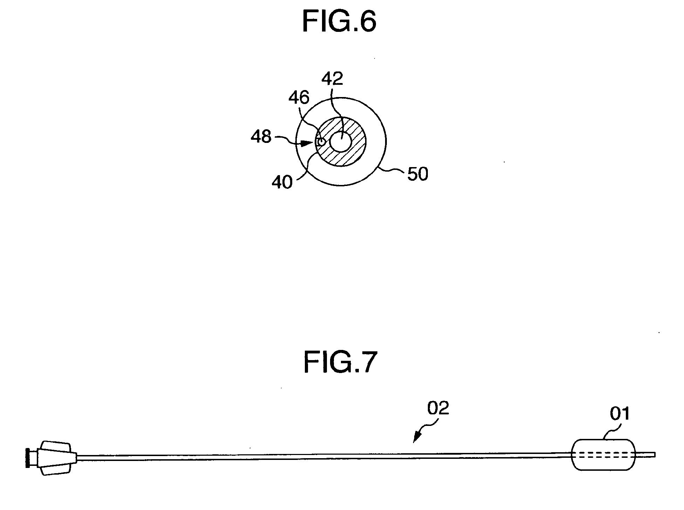

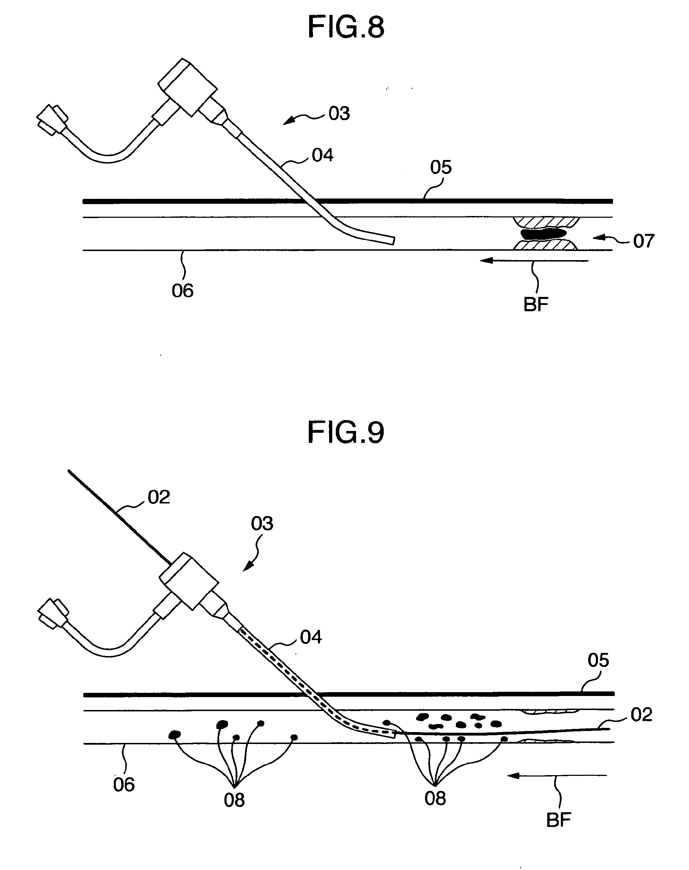

When a blood vessel is subjected to a percutaneous angioplasty treatment with regard to a stenosed or occluded part of the blood vessel, a balloon catheter (PTA catheter) 02 is used, which is provided with a balloon 01 for expanding the blood vessel (see Fig. 7 showing an inflatable/deflatable balloon 01 provided at a distal end of the catheter in an inflated state). When conducting the treatment with utilization of the catheter, a main conduit 04 of a multi-branched catheter-introducer tube 03 (for example, see Figs. 1 or 1A of JP-A-2001-508670) is inserted into the artery or the vein 06 through a patient's skin 05 (see Fig. 8 in which reference character BF indicates a blood flow direction). The catheter is inserted through the main conduit as far as a location corresponding to a diseased part in the blood vessel, and subsequently inflated to provide a dilation treatment to the stenosed or occluded part (see Fig. 9).

While the main conduit of the multi-branched catheter-introducer tube is inserted into the blood vessel against the blood flow BF, and the balloon of the catheter is inflated, which balloon is passed through the diseased part in the blood vessel, debris 08 of the hardened and stenosed diseased part destructed by the inflated balloon are partially allowed to flow downstream according to the blood flow. The debris 08 may flow toward distal ends of extremities in the case of the artery, or toward a heart in the case of the vein, thereby occurring a new occlusion of the blood vessel during flowing of the debris.

In order to prevent such an adverse effect encountered in the vasodilator treatment, the following way is conceived: A second multi-branched catheter-introducer tube is used and inserted into the blood vessel at a location downstream from the vasodilator treatment position in the direction of the blood flow. A second catheter is inserted through the second multi-branched catheter-introducer tube, and a second balloon mounted on a distal end of the second catheter is inflated to block the blood flow. However, this way have various problems which are a necessity of the two multi-branched catheter-introducer tubes and the two catheters each having a balloon, complicated treatment operations necessitating a lot of time, a difficult recovery of debris generated by destruction of a thrombus or stenosed diseased part, which debris are caught by the second blood flow stopper balloon, and a large physical burden on the patient due to the two inlets for insertion of the two multi-branched catheter-introducer tubes into the blood vessel.

### BRIEF SUMMARY OF THE INVENTION

Thus, an object of the present invention is to provide a multi-branched catheter-introducer tube, which can solve the above problems in the vasodilator treatment.

According to the invention, there is provided a multi-branched catheter-introducer tube used in a percutaneous intravascular treatment with utilization of a catheter, which comprises:
a check valve structure;
a main conduit connected to the check valve structure and adapted to be inserted into a blood vessel with its distal end;
a first port tube connected in communication to an inner chamber of the check valve structure; and
a second port tube connected to the check valve structure, wherein
the main conduit has two separate bores consisting of a first guide bore and a second guide bore therein, and an inflatable/deflatable blood flow stopper balloon mounted on an outer periphery of the main conduit at a portion closer to a distal end of the main conduit,
the blood flow stopper balloon is adapted to be inflated or deflated by a pressure of a control fluid supplied thereinto through the second port tube and the second guide bore,
the first port tube is used to draw floating debris in the blood vessel thereinto through the inner chamber, the first guide bore and an opening in the distal end of the main conduit,
the first guide bore is used as a bore for guiding the catheter into the blood vessel, through which the catheter inserted from an inlet of the check valve structure via the inner chamber is passed, and
the second port tube and the second guide bore are in communication with a passage provided in the check valve structure separately from the inner chamber.

Particular embodiments of the multi-branched catheter-introducer tube are as follows:
(1) The check valve structure comprises a housing defining the inner chamber; and an one-way valve which is openably/closably mounted on the opposite side of the inner chamber from the first guide bore to close the inlet of the inner chamber; wherein the first port tube and the first guide bore communicate with the inner chamber; and the second port tube communicates with the second guide bore via the passage formed in a housing wall defining the inner chamber.
(2) The second guide bore is formed in a thickness of a wall defining the first guide bore of the main conduit.
(3) An outlet port for the second guide bore is formed in an outer peripheral surface of the main conduit surrounded by the blood flow stopper balloon.
(4) The main conduit is made of a flexible material.
(5) The passage in the check valve structure communicating with the second port tube and the second guide bore can be formed within a wall of the check valve structure defining the inner chamber, but optionally can be provided as a passage tube along the outside of a body of the check valve structure.
(6) The main conduit can be made of, for example, polyester, polyvinyl chloride, stainless steel and so on.

Here, there will be provided below a description concerning operations of the multi-branched catheter-introducer tube according to the invention:
(1) Insertion of the main conduit: The multi-branched catheter-introducer tube is prepared, and the main conduit extending from the check valve structure is inserted into a blood vessel as far as a stenosed or occluded part, i.e., a diseased part in the blood vessel, in a direction from a downstream side to an upstream side (the diseased part side) of a blood flow.
(2) Inflation of the blood flow stopper balloon:
   The pressurized control fluid is fed through the second port tube connected to the check valve structure and through the second guide bore into the blood flow stopper balloon which is in a deflated state and which is in a position closer to the distal end of the main conduit (e.g., a position 1 to 2 mm spaced apart from a distal end), thereby inflating the blood flow stopper balloon. The thus inflated blood flow stopper balloon is brought into contact with an inner wall of the blood vessel to stop the blood flow.
(3) Insertion of a balloon catheter (PTA catheter): The balloon catheter is inserted through the inlet of the check valve structure into the inner chamber and further through the first guide bore in the main conduit into the blood vessel. The length of insertion of the balloon catheter into the blood vessel corresponds to a distance such that a balloon applied to a leading end of the balloon catheter reaches a position corresponding to the diseased part in the blood vessel. Then, the balloon of the balloon catheter is inflated at a location corresponding to the stenosed or occluded part in the blood vessel, which is the diseased part, thereby conducting an angioplasty. At this time, debris are released into the blood in the blood vessel, which debris are those generated from the diseased part (i.e. a thrombus or stenosed part) destructed by the balloon.
(4) Removal of floating debris: A negative pressure is introduced into the blood vessel through the inner chamber in the check valve structure, the first guide bore and the opening in the distal end (the leading end) of the main conduit, thereby causing debris of the thrombus or hardened/stenosed diseased part released by the inflation of the balloon (angioplasty treatment) to be drawn and discharged together with the blood.

The multi-branched catheter-introducer tube according to the present invention provides the following advantages:
(1) Since the inflatable/deflatable blood flow stopper balloon is mounted on an outer periphery of the main conduit at the portion closer to the distal end of the main conduit extending from the check valve structure, when debris produced by destruction of a thrombus part or a hardened and stenosed part (i.e. a diseased part) are released into the blood in the blood vessel during conducting the angioplasty treatment with utilization of a balloon catheter introduced into the blood vessel through the main conduit, which destruction is made by a balloon mounted on the balloon catheter, by inflating the blood flow stopper balloon which is inserted into the blood vessel through the main conduit, the debris can be drawn and discharged together with the blood by introducing the negative pressure into the blood vessel through the first port tube, the inner chamber in the check valve structure, the first guide bore in the main conduit and the opening in the distal end of the main conduit. Thus, the introduction of the balloon catheter into the blood vessel and the removal of the debris of the diseased part released in the blood flow by the vasodilator treatment can be achieved with utilization of the single multi-branched catheter-introducer tube. Therefore, it is possible to simplify the operations in the vasodilator treatment, to shorten the time required for the treatment, to alleviate the physical burden on the patient, including a reason that the appliances are inserted into the blood vessel at only one point, and to reduce the cost for the treating appliances.
(2) The main conduit has the first and second guide bores therein, which are the two separate guide bores, and the blood flow stopper balloon mounted at the portion closer to the distal end of the main conduit can be inflated by the pressure of the control fluid supplied through the second port tube and the second guide bore.
(3) Since the second guide bore can be formed in the range of the wall thickness of the wall defining the first guide bore of the main conduit, it is possible to facilitate the insertion of the main conduit into the blood vessel by forming the main conduit to have a circular cross-sectional shape. Alternatively, it is also possible to integrate two tubes to produce the main conduit having the first and second guide bores.

### BRIEF DESCRIPTION OF THE SEVERAL VIEWS OF THE DRAWINGS

Fig. 1 is a schematic view for illustrating a percutenous intubation angioplasty with utilization of a multi-branched catheter-introducer tube as one embodiment of the present invention;
Fig. 2 is a partially broken side view of the multi-branched catheter-introducer tube shown in Fig. 1;
Fig. 3 is a view indicated by arrows III-III in Fig. 2;
Fig. 4 is a cross sectional view of the multi-branched catheter-introducer tube taken along a line IV-IV in Fig. 2;
Fig. 5 is a side view of the multi-branched catheter-introducer tube shown in Fig. 2, in which a blood flow stopper balloon is expanded;
Fig. 6 is a cross sectional view taken along a line VI-VI in Fig. 5;
Fig. 7 is a side view of a balloon catheter (i.e. a PTA catheter) as a prior art example;
Fig. 8 is a schematic view showing a multi-branched catheter-introducer tube as a prior art example, in which a main conduit is inserted into a blood vessel; and
Fig. 9 is a schematic view showing the balloon catheter inserted into a blood vessel with utilization of the multi-branched catheter-introducer tube as the prior art example.

### DETAILED DESCRIPTION OF THE INVENTION

Now a particular embodiment of the present invention shown in the drawings will be described.

Fig. 1 schematically shows a state in which a percutaneous intubation angioplasty is being carried out with utilization of a multi-branched catheter-introducer tube 10 as one embodiment of the present invention. While the shape and structure of the multi-branched catheter-introducer tube 10 will be described later, a balloon catheter (a catheter shaft S) is shown as having been inserted into a blood vessel BV with utilization of the multi-branched catheter-introducer tube 10. A main conduit 40 of the multi-branched catheter-introducer tube 10 has been inserted into the blood vessel against a blood flow BF, and as a result of a vasodilator treatment by the balloon catheter, floating debris TH such as thrombus debris have been released into the blood flow from a diseased part to flow toward the main conduit 40. However, since a blood flow stopper balloon 50 provided on a distal end of the main conduit 40 has been inflated to block the inside of the blood vessel, the floating debris TH cannot flow downstream. In Fig. 1, a reference character SK denotes a skin of the patient.

The multi-branched catheter-introducer tube 10 comprises main components which are a check valve structure 20, the main conduit 40 connected to the check valve structure 20, the blood flow stopper balloon 50 applied to an outer periphery of the main conduit 40 at a portion closer to the distal end thereof, a first port tube 60 connected to the check valve structure 20, and a second port tube 70 connected to the check valve structure 20.

### Check valve structure 20

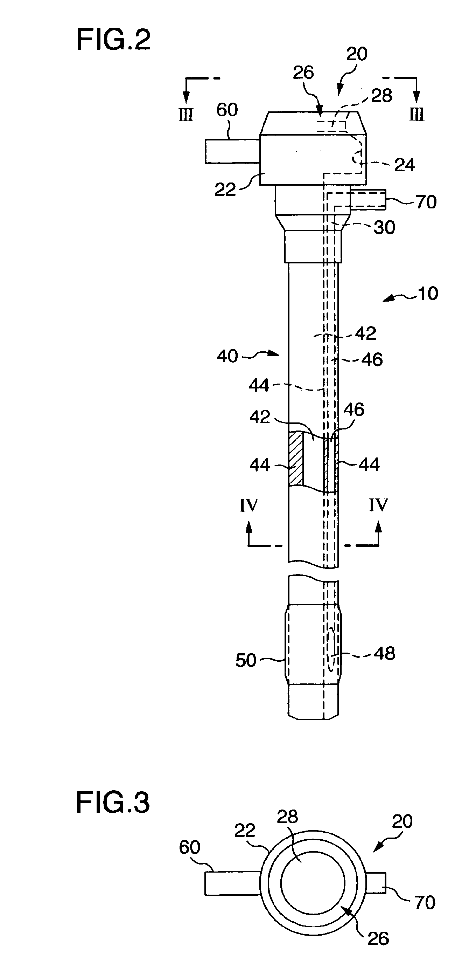

The check valve structure 20 will be described below with reference to Figs. 2, 3 and 5.

The check valve structure 20 has a body which is a housing 22. The housing 22 defines an inner chamber 24 and opens at its top to form an inlet 26 for the inner chamber 24. An one-way valve 28 is openably/closably mounted in the inlet 26 and adapted to be opened exclusively into the inner chamber 24.

A bore in the first port tube 60 connected to the housing 22 communicates with the inner chamber 24. A bore in the second port tube 70 likewise connected to the housing 22 communicates with a passage 30 formed in a housing wall defining the inner chamber 24.

### Main conduit 40

As shown in Figs. 2 and 4 to 6, the main conduit 40 connected to the check valve structure 20 is a cylindrical tube and includes a first guide bore 42 which is a central tube bore communicating with the inner chamber 24 in the housing 22, and a second guide bore 46 formed in a tube wall 44. The second guide bore 46 communicates with the passage 30 formed in the housing wall of the housing 22 and further communicates with the bore in the second port tube 70. The second guide bore 46 opens in an outer peripheral surface of the main conduit 40 at a balloon-applied portion closer to the distal end of the main conduit 40 to provide a balloon-controlling opening 48.

### Blood flow stopper balloon 50

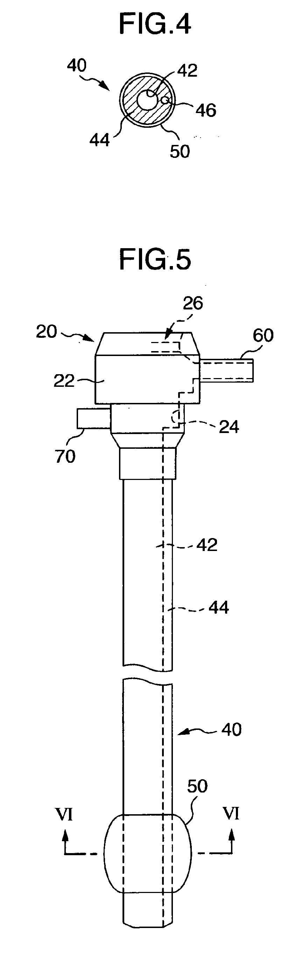

As shown in Figs. 2 and 4 to 6, the blood flow stopper balloon 50 formed of a flexible material (such as a natural rubber) is mounted on a portion closer to the distal end of the main conduit 40 to cover the balloon-controlling opening 48 and to surround the outer peripheral surface of the main conduit 40. The blood flow stopper balloon 50 is a cylindrical member (a sleeve form member) and secured hermetically at both opposite ends thereof to the main conduit 40. Therefore, when a pressurized fluid (i.e. a control fluid) is fed into the blood flow stopper balloon 50 through the balloon-controlling opening 48 in a state shown in Figs. 2 and 4, the blood flow stopper balloon 50 is expanded (or inflated) into a state shown in Fig. 5 and 6. An expansion volume of balloon is so determined that the blood flow stopper balloon 50 can be brought into contact with an inner surface of the blood vessel to block the blood flow.

While the multi-branched catheter-introducer tube 10 is constructed as described above, details of operation thereof will be described below. Since the outline of the percutaneous intubation angioplasty has been described in the introductory part of the description of the embodiment, here the operation of the multi-branched catheter-introducer tube 10 will be detailed.
(1) Introduction of the catheter into the blood vessel: When the main conduit 40 of the multi-branched catheter-introducer tube 10 is inserted into the blood vessel, the blood entered into the main conduit 40 and the inner chamber 24 of the housing 22 does not flow out by virtue of the one-way valve provided to the inlet 26 of the check valve structure 20. The insertion of the catheter into the check valve structure 20 is conducted through the one-way valve 28. The inserted catheter is advanced via the inner chamber 24 of the housing 22 into the second guide bore 42 in the main conduit 40 and out of the distal end of the main conduit 40 so as to enter into the blood vessel.
(2) Inflation and deflation of the blood flow stopper balloon 50: A fluid-controlling syringe which is not shown is fitted into an outer end 72 of the second port tube 70, and a pressurized controlling fluid is supplied into the blood flow stopper balloon 50 through the second port tube 70, the passage 30 in the housing wall, the second guide bore 46 in the main conduit 40 and the balloon-controlling opening 48 by the operation of the syringe. The supply of the pressurized controlling fluid causes the blood flow stopper balloon 50 to be expanded (or inflated). After completion of the angioplasty treatment, a negative pressure is applied to the fluid path by the operation of the syringe, thereby discharging the fluid out of the blood flow stopper balloon 50 to deflate it.
(3) Treatment of floating debris TH: It has been described previously that as a result of the vasodilator treatment by the balloon catheter, the floating debris TH such as thrombus debris have been released into the blood flow from a diseased part to float in the blood flow toward the main conduit 40. The floating debris TH are prevented from further flowing downstream by the blood flow stopper balloon 50, but it is necessary to remove the floating debris TH to the outside after completion of the vasodilator treatment. For this purpose, a fluid-controlling syringe (not shown) is fitted into the outer end 62 (Fig. 1) of the first port tube 60. When the syringe is operated to draw the blood existing the upstream side of the blood flow stopper balloon 50 and also the blood existing within the inner chamber 24 and the first guide bore 42 into the syringe, the floating debris TH can be removed out of the blood vessel.

## Claims

1. A multi-branched catheter-introducer tube (10) used in a percutaneous intravascular treatment with utilization of a catheter (S), which comprises a check valve structure (20);
a main conduit (40) connected to the check valve structure (20) and adapted to be inserted into a blood vessel (BV) with its distal end;
a first port tube (60) connected in communication to an inner chamber (24) of the check valve structure (20); and
a second port tube (70) connected to the check valve structure (20), wherein
the main conduit (40) has two separate bores (42, 46) consisting of a first guide bore (42) and a second guide bore (46), and an inflatable/deflatable blood flow stopper balloon (50) mounted on an outer periphery of the main conduit (40) at a portion closer to a distal end of the main conduit (40),
the blood flow stopper balloon (50) is adapted to be inflated or deflated by a pressure of a control fluid supplied thereto through the second port tube (70) and the second guide bore (46),
the first port tube (60) is used to draw floating debris (TH) in the blood vessel (BV) thereinto through the inner chamber (24), the first guide bore (42) and an opening in the distal end of the main conduit,
the first guide bore (42) is used as a bore for guiding the catheter (S) into the blood vessel (BV), through which the catheter (S) inserted from an inlet (26) of the check valve structure (20) via the inner chamber (24) is passed, and
the second port tube (70) and the second guide bore (46) are in communication with a passage provided in the check valve structure (20) separately from the inner chamber.

2. A multi-branched catheter-introducer tube (10) according to claim 1, wherein the check valve structure (20) comprises a housing (22) defining the inner chamber (24); and an one-way valve (28) which is openably/closably mounted on the opposite side of the inner chamber (24) from the first guide bore (42) to close the inlet (26) of the inner chamber (24), and
wherein the first port tube (60) and the first guide bore (42) communicate with the inner chamber (24), and the second port tube (70) communicates with the second guide bore (46) via the passage (30) formed in a housing wall defining the inner chamber (24).

3. A multi-branched catheter-introducer tube (10) according to claim 1 or 2, wherein the second guide bore (46) is formed in a thickness of a wall (44) defining the first guide bore (42) of the main conduit (40).

4. A multi-branched catheter-introducer tube (10) according to any one of claims 1 to 3, wherein an outlet port (48) for the second guide bore (46) is formed in an outer peripheral surface of the main conduit (40) surrounded by the blood flow stopper balloon (50).

5. A multi-branched catheter-introducer tube according to any one of claims 1 to 4, wherein the main conduit (40) is made of a flexible material.
